# EUROPEAN PATENT SPECIFICATION

(11) **EP 1 979 086 B1**
(45) Date of publication and mention of the grant of the patent: **02.11.2011**
(21) Application number: 06831524.1
(22) Date of filing: 22.12.2006
(51) Int. Cl.: B01J 19/00, B01L 3/00

(54) **FLOW-TYPE LABORATORY OZONOLYSIS APPARATUS AND METHOD OF PERFORMING OZONOLYSIS REACTION**
LABOROZONOLYSEAPPARATUR VOM STRÖMUNGSTYP UND VERFAHREN ZUR DURCHFÜHRUNG EINES OZONOLYSEREAKTION
APPAREIL D'OZONOLYSE DE LABORATOIRE DE TYPE À ÉCOULEMENT ET PROCÉDÉ PERMETTANT DE METTRE EN OEUVRE UNE RÉACTION D'OZONOLYSE

(30) Priority: 23.12.2005 HU 0501206
(43) Date of publication of application: 15.10.2008
(73) Proprietor: Thalesnano Nanotechnológiai Zrt., 1031 Budapest (HU)
(72) Inventor: SZALAY, Dániel, H-1062 Budapest (HU); VARGA, Norbert, H-2800 Tatabánya (HU); BONCZ, Ferenc, H-1133 Budapest (HU); DARVAS, Ferenc, H-1016 Budapest (HU); KARANCSI, Tamás, H-1124 Budapest (HU); GÖDÖRHÁZY, Lajos, H-2030 Érd (HU); ÜRGE, László, H-1029 Budapest (HU)
(74) Representative: Szabo, Zsolt
(86) International application number: PCT/HU2006/000125
(87) International publication number: WO 2007/072097

(56) References cited:
- EP-A2- 1 203 954
- WO-A-02/41995
- WO-A-2005/107936
- US-A- 4 185 025
- US-A1- 2005 133 457
- US-B1- 6 512 131
- EHRFELD W ET AL: "Microreactors - New Technology for Modern Chemistry, PASSAGE" MICROREACTORS : NEW TECHNOLOGY FOR MODERN CHEMISTRY, WEINHEIM : WILEY, 2000, pages 6-13, XP002384564 ISBN: 3-527-29590-9

## Description

The present invention relates to a flow-type laboratory scale ozonolysis apparatus comprising a liquid reservoir, a feed pump, a uniting element with two inlets and an outlet, a reactor unit and a pressure-adjusting means, all connected into a flow path, the apparatus further comprising an ozone source and a dispensing valve transmitting a gas stream only in a single direction and being installed between the ozone source and one of the inlets of the uniting element. The invention also relates to a method of performing ozonolysis reaction of a given substance.

In the chemical industry, the term "ozonolysis", in general, refers to the oxidation of organic hydrocarbon compounds (in particular, of alkenes and aromatic compounds) accompanied by a chain scission. Ozonolysis reactions represent chemical reactions that are relatively rarely applied. Due to their hazardous nature, the ozonolysis reactions are not spread in the field of industry, agriculture and therapy, however, they are widely used for disinfection and sewage treatment. The ozonolysis reactions usually comprise of two reactions that take place/are carried out one after the other. The exothermic reaction, accompanied by a strong generation of heat, of the organic substance and the ozone mixed thereto takes place within a primary reaction. In a secondary reaction following the primary one, a further reaction (preferentially decomposition or stabilization) of the intermediate (the so-called ozonide) produced in the primary reaction takes place via adding an additive thereto. To accomplish ozonolysis reactions, addition of gaseous ozone in a great amount is required. Due to the high explosion risk and reactivity of the intermediate formed, the implementation of ozonolysis reactions on the industrial scale is, however, possible only in that case if safety means of relatively high costs are applied and proper safety measures are adhered to. Furthermore, the production of gaseous ozone in a necessary amount requires the usage of a high performance ozonizer that also has large physical dimensions.

European Patent No. 1,039,294 A2 discloses an ozone hydroxyl radical analyzer and a chemical assaying method accomplished by the analyzer. Said analyzer comprises a reaction channel with an inlet and an outlet accommodating an ozonolysis reaction, a feed pump for introducing a sample through the inlet into the reaction channel to be analyzed by means of an ozonolysis reaction, an ozone generator feeding an ozone containing gas through the inlet into the reaction channel and a control unit. The ozone generator, by the nature thereof, is built up of concentric tubular electrodes, on which high voltage is applied when is operated. The ozone consumed in the reaction is generated by passing an oxygen containing gas through the electrodes carrying high voltage. A given amount of sample fed into the reaction channel is subjected to the assay by the analyzer which means that the analyzer is a batch-type analyzer.

German Pat. Publication No. 199 10 639 A1 discloses an apparatus for cleaning spent water, to be used preferably in a car washer, having an ozone generator in the form of an electrolysis cell operated on electricity. The apparatus can be equally put into a continuously circulated stream of water or into the water led out from the stream and collected in a reservoir. The chemical reaction between the contamination content of the spent water and the ozone that results in the purification of water is performed within a reservoir of large dimensions storing said spent water to be treated by bubbling the ozone produced in the electrolysis cell sunk into the spent water to the spent water and then by continuously rebubbling the not reacted ozone reaching the water surface from a volume of the reservoir being above the water surface back into the water.

International Publication No. WO01/40124 discloses a method and an industrial scale apparatus for treating electronic components, such as e.g. integrated circuits, with an ozonated liquid substance. Said apparatus comprises a vessel for accommodating and storing a stock fluid, an ozone source in fluid communication with the latter, an exhaust for discharging the ozonated stock fluid from the vessel, and a back-pressure regulator unit for regulating pressure within the vessel and for controlling opening/closing of the exhaust. The ozonated fluid is produced within the vessel by means of bubbling the ozone generated by the ozone source into the liquid. To enhance efficiency of the treatment, before the liquid exits through the exhaust the size of the ozone bubbles of the ozonated liquid is decreased to at most about 50 µm by means of installing a suitably perforated member. However, no heterogeneous phase ozonolysis reaction takes place within the apparatus.

The above discussed solutions are either based on industrial scale apparatuses or not flow-type ones. Furthermore, the known solutions can be neither used during the discovery/compound finding phases of pharmaceutical research.

International Publication Pamphlet No. WO2005/107936, being the closest prior art to the present invention, teaches a flow-type laboratory-scale apparatus and also a process for carrying out hydrogenation reactions of fluid-phase samples. The apparatus comprises a reservoir, a feed pump in the form of a liquid pump of a constant volume rate, a collecting element with two inlets and an outlet, a reactor for carrying out hydrogenation of a sample within its inner space and a pressure-adjusting unit provided with an electrical control and arranged downstream of the reactor. The apparatus also comprises a hydrogen source, as well as a valve transmitting the hydrogen gas stream only into a single direction and connected between the hydrogen source and the second inlet of the collecting element The reactor is formed as a replaceable cartridge reactor comprised of a single reaction zone with a packing that increases the flow resistance and facilitates the mixing of the liquid-phase sample and the gaseous hydrogen fed into the reaction zone. The core feature of the hydrogenating process is that said hydrogenation reaction of the sample takes place within the reaction zone in the presence of an appropriate catalyst and in a given pressure range maintained by the pressure-adjusting unit, without a need for further intervention into the system.

U.S. Pat. No. 6,512,131 B1 discloses a continuously operated counter tube reactor and a process for carrying out a multi-phase reaction in particular between a fluid phase reactant flowing into a first direction (i.e. running down) and a continuous gaseous phase reactant flowing into a second direction which is opposite to the first direction (i.e. flowing up). To produce the gaseous phase reactant, which is an ozone-oxygen mixture, external ozone generators of the Siemens-type (based on corona discharge) fed by pure oxygen are applied. Furthermore, pure oxygen is provided by further external sources.

U.S. PaL No. 4,185,025 relates to a system of separate reactors wherein both of the reactants, consisting on the one hand of an organic charge mixture to be ozonized (i.e. the fluid phase reactant) and on the other hand of ozonic gas (i.e. the gaseous phase reactant), are fed in parallel flow through at least two counterflow-connected reactors. Here, the gaseous phase reactant, that is the ozonic gas, is produced by an external ozone generator, preferably by silent electrical discharge at high voltages using air as the required oxygen source.

In view of the above it is apparent that there would be a great need for developing an ozonolysis apparatus that subjects tiny amounts of a chemical substance to ozonolysis reaction in a controlled way and by maintaining it in a continuous flow.

In light of the above, the object of the present invention is to develop a flow-type ozonolysis apparatus capable of accomplishing the aimed ozonolysis reaction in a safe and convenient manner, as well as with a high reaction yield as a consequence of the amount of reactants being involved in the ozonolysis reaction carried out therein in a controlled manner. A further object of the present invention is to provide an ozonolysis apparatus that can be cheaply manufactured and hence widely used in the laboratory practice due to the low manufacturing costs of its major components, that is the ozone source and the reactor unit themselves. A yet further object of the present invention is to provide a safe process for ozonolysis reactions. Further objects of the present invention will be apparent from the detailed discussion of the solution according to the invention.

In one aspect of the present invention, the above objects are achieved by a flow-type laboratory scale ozonolysis apparatus, according to claim 1 wherein the feed pump is a liquid pump generating a constant volume rate, the liquid reservoir contains at least the substance, as a solute, to be subjected to the ozonolysis reaction and the reactor unit consists of first and second reactor zones being different as to their function, wherein the outlet of the first reactor zone is connected to the inlet of the second reactor zone in the flow path and a substance intake is inserted into the flow path between the reactor zones, and the pressure-adjusting means is connected into the flow path after the reactor unit and is provided with an electrically governed control.

Preferred embodiments of the apparatus according to the present invention are defined by dependent claims 2 to 17.

In a further aspect of the present invention, the above objects are achieved by providing a method of performing laboratory scale ozonolysis reaction of a given substance, according to claim 18 comprising the steps of supplying a given amount of substance being solved to be subjected to ozonolysis reaction by means of a feed pump into a flow path; feeding ozone through a dispensing valve into the flow path in a section located after the substance supplying position; leading solved substance through a reactor unit comprising first and second reactor zones arranged in a section of the flow path located after the ozone feeding position; supplying an additive needed for the completion of ozonolysis reaction into the flow path after the first reactor zone of the reactor unit; maintaining the pressure of the reaction in a given pressure range by means of a pressure adjusting means arranged in the flow path after the reactor unit; collecting the product generated in the second reactor zone of the reactor unit in a product receptacle connected to the end of the flow path.

Preferred embodiments of the method according to the invention are specified by dependent claims 20 to 22.

The invention will now be described in detail with reference to the accompanying drawing, wherein
- Figure 1 shows a schematic block diagram of an embodiment of the laboratory scale ozonolysis apparatus;
- Figure 2A shows a sectional view of a possible embodiment of an ozone generating electrolysis cell used within the ozonolysis apparatus according to the invention;
- Figure 2B illustrates schematically the electrode structure of the ozone generating electrolysis cell of Figure 2A;
- Figure 3A shows a sectional view of a possible embodiment of a microfluidic reactor to be preferentially used within the ozonolysis apparatus according to the invention; and
- Figure 3B illustrates a sectional view along the line A-A of the microfluidic reactor shown in Figure 3A.

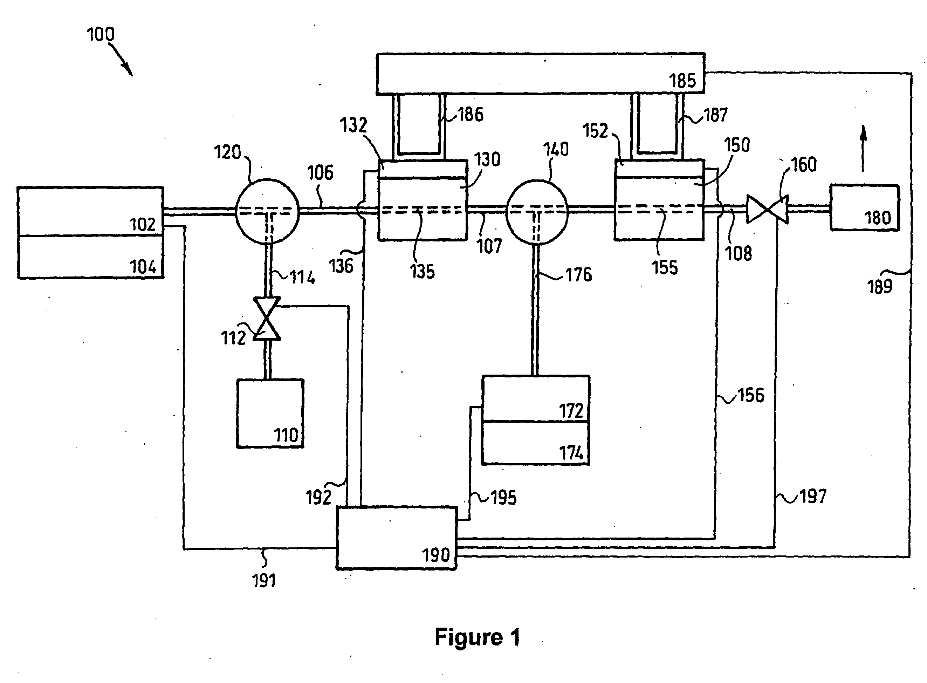

Referring now to Figure 1, ozonolysis apparatus 100 schematically shown in Figure 1 comprises a liquid reservoir 104 equipped with a feed pump 102, uniting elements 120, 140, a reactor unit comprising a first reactor 130 with a reaction channel 135 and a second reactor 150 with a reactor channel 155, a pressure-adjusting means 160, a product receptacle 180, a control unit 190, a dispensing valve 112 and an ozone source 110. The ozonolysis apparatus 100 further comprises a liquid reservoir 174 equipped with a feed pump 172. The inlet of the feed pump 172 is in flow communication with the liquid reservoir 104, while its outlet is connected to a first inlet of the uniting element 120 inserted into a pipe 106. Through a pipe 114 and the dispensing valve 112 inserted into the pipe 114, the ozone source 110 is connected to a second inlet of the uniting element 120. The outlet of the uniting element 120 is connected to the inlet of the reaction channel 135 of the first reactor 130. The outlet of the reaction channel 135 of the first reactor 130 is connected to a first inlet of the uniting element 140 inserted into a pipe 107. To a second inlet of the uniting element 140 the outlet of the feed pump 172 is connected, optionally through a pipe 176. The inlet of the feed pump 172 is in flow communication with the liquid reservoir 174. The outlet of the uniting element 140 is connected to the inlet of the reaction channel 155 of the second reactor 150. The outlet of the reaction channel 155 of the second reactor 150 opens into the product receptacle 180 through a pipe 108 and the pressure-adjusting means 160 inserted into the pipe 108. As a result of connecting the specified elements to each other, the ozonolysis apparatus 100 will be possessed of a continuously connected flow path running from the outlet of the feed pump 102 to the inlet of the product receptacle 180.

Through leads 191, 192, 195, 197, the programmable control unit 190 is in electrical connections with the feed pump 102, the dispensing valve 112, the feed pump 172 and the pressure-adjusting means 160, respectively.

The liquid reservoir 104 contains the substance, and/or its solution (from now on, referred to as sample solution in brief) to be subjected to an ozonolysis reaction. If performing the ozonolysis reaction requires the presence of a catalyst, the catalyst, in a suitable form, is mixed to the sample solution, and hence it is also present within the liquid reservoir 104. Feeding of the sample solution (optionally also containing the catalyst) into the flow path is performed by the feed pump 102. The feed pump 102 is preferably provided by a HPLC pump that is adapted for maintaining a continuous flow of the sample solution with a constant flow rate. Actually, the HPLC pump is a precision pump that operates with a constant feed rate, set as desired, in the presence of the pressure created and continuously maintained by the pressure-adjusting means 160; the feed rate can be, of course, arbitrarily varied.

The liquid reservoir 174 contains the additive, that is, the substance and/or a solution thereof (from now on, referred to as solution of additive in brief) needed for the decomposition/stabilization of the intermediate produced in the ozonolysis reaction. If performing a second reaction needed for decomposing/stabilizing the intermediate requires the presence of a catalyst, the catalyst, in a suitable form, is mixed to the solution of additive, and hence it is also present within the liquid reservoir 174. Feeding the solution of additive (optionally also containing the catalyst) into the flow path is performed by the feed pump 172. The feed pump 172 is preferably also provided by a HPLC pump that is adapted for maintaining a continuous flow of the solution of additive with a constant flow rate. The feed rate provided by the HPLC pump can be, of course, also arbitrarily varied.

The uniting elements 120, 140 are preferably T-shaped elements that are made of a plastic material with a chemical resistivity against reactive ozone, preferably a fluorinated and/or chlorinated polymer, more preferably polytetrafluoroethylene (PTFE). In a possible further embodiment, only the inner surfaces of the flow channels of the uniting elements 120, 140 are made of or coated with the chemically resistant plastic material. To facilitate the diffusion limited ozonolysis reaction taking place within the first reactor 130, the uniting element 120 blends the sample solution and the pressurized ozone entering through the inlets thereof. A sample solution with ozone bubbles exits through the outlet of the uniting element 120. The uniting element 140 blends the intermediate produced within the first reactor 130 and the additive taking part in a second reaction required to decompose or stabilize the intermediate itself. The uniting elements 120, 140 can be, of course, formed as any other members that ensure the proper mixing of the liquid and gaseous reactants fed into the uniting elements 120, 140.

The dispensing valve 112 on the one hand restrains the backflow of the sample solution to the ozone source 110 and on the other hand performs the feeding of ozone. The dispensing valve 112 is preferably an electronically controlled on/off switch valve preferentially made of PTFE that is actuated by the control unit 190. As far as the efficiency of the diffusion limited reaction between the sample solution and the ozone is concerned, the size of the contact surface between the reactants is of the greatest importance. Accordingly, the dispensing valve 112 is designed to dispense bubbles of the least possible size, preferably with the size of about 10 µl, that is, microbubbles. If the size of the ozone bubbles dispensed by the dispensing valve 112 is too large from the point of view of the reaction planned, an end sheet (not shown in the drawing) preferably made of PTFE with perforations of desired diameter is arranged at the ozone access inlet of the uniting element 120 to decrease the size of the ozone bubbles being mixed into the sample solution.

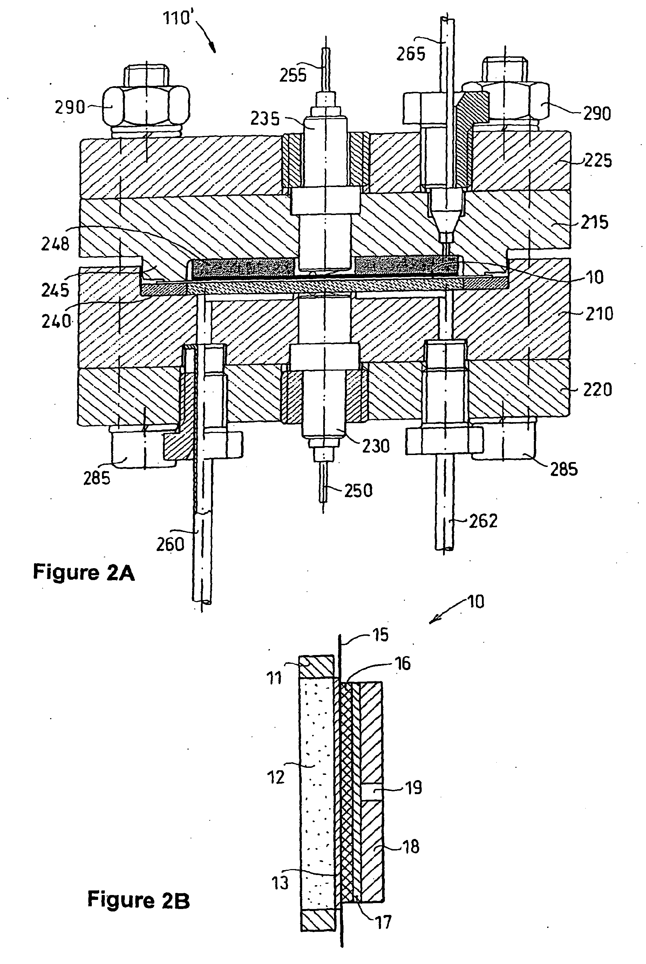

As is well-known, gaseous ozone is of extraordinary hazard, its handling, in general, requires the usage of special means and adherence to proper safety measures. Accordingly, in the present solution the ozone gas consumed in the ozonolysis reaction is generated preferentially in-situ: the ozone is obtained preferably from water by means of electrolysis (i.e. by decomposition of water). Accordingly, in one of its preferred embodiments the ozone source 110 is formed as one or more asymmetric pressure ozone generating electrolysis cells. Figure 2A illustrates a preferred embodiment of the ozone source 110 used in the ozonolysis apparatus 100 according to the invention, formed as an ozone generating electrolysis cell 110'. The electrode structure 10 of Figure 2B used in the cell 110' comprises a cathode 13, an ozone generating anode 16, a proton exchange membrane 15 arranged therebetween and a first electrode support 17 arranged on a side of the anode 16 located opposite to the membrane 15. The electrode support 17 is arranged on an (anode side) bearing member 18 provided with a through-hole 19 for an electrical contact. The cathode 13 is formed on a second (cathode side) electrode support 12 arranged in a (cathode side) bearing member 11.

The electrode support 12 serves on the one hand for providing electrical contact between e.g. the DC power supply of the ozonolysis apparatus 100 and the cathode 13 and on the other hand for directing the water required for the electrolysis to the cathode 13 during the operation of the cell and diverting the produced hydrogen gas from the cathode 13. Accordingly, the electrode support 12 is formed as a member with high electrical conductivity and a porous structure, as well as with high mechanical strength in order to tolerate the high pressures of up to 20 bars that may develop inside the 110' cell. In particular, the electrode support 12 is a thin and porous titanium frit arranged in the bearing member 11 and produced by high-pressure cold moulding of titanium granulate. Here and from now on, by the term "frit" a material made of a powder of grains by cold moulding is referred to. The titanium granulate preferably comprises three different sizes of titanium grains in a layered structure, in which the layers are arranged in the order of the grain size in such a way that before moulding, a relatively coarse-grained titanium powder (preferably comprising grains having a dimension of 600-1200 µm) is put into the bearing member 11 to the bottom, then a titanium powder of medium sized grains (preferably comprising grains having a dimension of 350-600 µm) is applied thereon, and finally, a fine-grained titanium powder (preferably comprising grains having a dimension of 150-350 µm) is applied thereon. Hence, the titanium frit produced by moulding and the cathode side electrode support 12 made therefrom will have a grain size gradient in the direction of depth.

The cathode side bearing member 11 is made of a special, chemically resistant plastic shaped to e.g. an annular member. It is obvious, however, that the bearing member 11 may be made of any other material and may have any other shape as well.

An essential condition for the efficient cell operation is the good electrical contact between the cathode 13 and the anode 16, as well as the membrane 15. Therefore, formation of the cathode 13 on the electrode support 12 made of the titanium frit has key importance. In the electrode structure 10 of Figure 2B, it is preferred that for the cathode 13, extra fine-grained platinum powder (so called platinum black) is used.

The proton exchange (or proton conducting) membrane 15 is preferably in the form of a sulphonylated, perfluorinated polymeric resin membrane, most preferably the polymeric membrane Nafion® of DuPont de Nemours, Co. The membrane 15 is the solid electrolyte of the cell 110'. In addition, the membrane 15 provides the separation of gases produced at the cathode and the anode. The water required for the electrolysis is introduced at one side of the membrane 15, through the second electrode support 12 provided with the cathode 13, whereas the gaseous mixture of oxygen and ozone to be used is generated on the other side of the membrane 15, that is, at the anode 16.

The anode 16 serves for supporting the anode side electrochemical reaction. For the anode 16, electrically conducting metals, semimetals and/or oxides thereof are used in general. The use of the oxides of transition metals is advantageous because those are commonly available and inexpensive. However, the mechanical strength of these oxides is low, thus they have to be placed on a substrate with high mechanical strength and chemical resistance against the highly corrosive gaseous mixture of oxygen and ozone so that said oxides could tolerate high pressures arising in the cell 110' during operation without being mechanically damaged.

For the electrode support 17 used to support the anode 16, noble metals (e.g. platinum) with good electrical conductivity or the alloys and/or the mixture thereof are used. The electrode support is formed as a suitably perforated platinum sheet provided with through-holes having preferably a diameter of at least 0.8 mm.

The anode side bearing member 18 serves for removing the gaseous mixture of oxygen and ozone generated at the anode 16 during operation of the 110' cell from the anode 16. The bearing member 18 is additionally used to clamp the electrode support 17 to the anode 16 and the latter to the membrane 15 in order to provide a perfect electrical contact, as well as a homogenous transition surface therebetween. In the electrode structure 10 shown in Figure 2B, the bearing member 18 is made of a resilient, porous, chemically resistive material, preferably of PTFE frit produced from grained PTFE by high-pressure moulding. The bearing member 18 is provided with a through-hole 19. In the assembled cell 110' the through-hole 19 is adapted to receive an anode side conducting member used for electrically connecting the anode side electrode support 17 and the power supply.

In the ozone generating cell 110' forming the ozone source 110 of the ozonolysis apparatus 100 the anode 16 is made of a material with good electrical conductivity, plasticity, high evolution potential and chemical resistance against the highly corrosive gaseous mixture of oxygen and ozone, preferably a mixture of lead dioxide and PTFE comprising PTFE in an amount of at least 10% by weight. The mixture of lead dioxide and PTFE is produced from solid-phase raw materials at ambient temperatures with no use of further additives. Before producing the anode 16, the lead dioxide constituting a first component of the mixture of lead dioxide and PTFE used as the material of the anode 16 is subjected to continuous crushing, which results in lead dioxide grains of colloid size, i.e. with an average grain size of 0.5-100 µm, produced from the initial macroscopic sized lead dioxide pieces. For the other component of the mixture of lead dioxide and PTFE used as the material of the anode 16, solid PTFE filaments having a fibrous (cotton wool-type) structure and a thickness of 50-100 µm, as well as a length of up to 1 mm are used. The PTFE filaments with such dimensions can be produced by abrasive machining or abrasion of a PTFE block.

In order to produce the material of the anode 16, lead dioxide crushed into grains of colloid size in an amount of e.g. about 1600 mg and PTFE in the form of fine elementary filaments in an amount of e.g. about 300 mg are strongly mixed together. After several, preferably 10 minutes of mixing, the thus obtained mixture is poured into a frit moulding tool prepared especially for this purpose and then pressed therein by applying a pressure of at least 50 MPa, preferably 250 MPa, to shape a sheet with a thickness of 0.25 mm. During the moulding process, the PTFE filaments get tangled and fused, causing the lead dioxide grains to be joined at the same time. The resulted lead dioxide/PTFE sheet has compact dimensions and a continuous surface, it can be easily formed mechanically, and in addition, it is resilient and ductile. Then the anode 16 is prepared by cutting the thus obtained lead dioxide/PTFE sheet to the desired size and shaping it.

When designing the construction of the cell 110' shown in Figure 2A and achieved by utilizing the electrode structure 10, and when selecting the materials for the cell 110', the chemical resistance against the gaseous mixture of oxygen and ozone and the mechanical strength due to the pressure of the gas generated by electrolysis of water are kept in view. The cell 110' in its assembled state is composed of a cathode side half cell 210 and an anode side half cell 215 that are fixed together in a form-fitting and thereby sealed manner. The electrode structure 10 is arranged in a seat 240 formed in the half cell 210 and defined by a bottom wall and a side wall, wherein the bearing member 11 of said electrode structure 10 (see Figure 2B) abuts on the bottom wall of the seat 240. The form-fitting abutment is established between the outer surface of a compressive flange 245 of the half cell 215 and the side wall of the seat 240. The half cell 215 is provided with a depression 248 for receiving the anode side of the electrode structure 10, wherein said depression 248 is laterally defined by the compressive flange 245. In the assembled cell 110', the bearing member 18 of the electrode structure 10 (shown in Figure 2B) is in close contact with the half cell 215 within the depression 248, whereas the compressive flange 245 pushes the electrode structure 10 to the bottom wall of the seat 240 with firmly fixing it thereby.

The cathode side half cell 210 is provided with through-holes (not referenced in the drawings) for receiving a water feeding connector 260, a hydrogen and water discharging connector 262 and a cathode side electrical connector casing 230 in a sealed manner. The anode side half cell 215 is provided with through-holes (not referenced in the drawings) for receiving an ozone/oxygen gas discharging connector 265 and an anode side electrical connector casing 235 in a sealed manner. The half cells 210, 215 are made of a chemically resistant, gas-proof material, preferably some kind of plastic, and formed preferably by injection moulding, machining or another shaping process.

There is at least one current conducting member 250 in the electric connector casing 230 arranged for providing electrical connection between the power supply and the cathode 13 (see Figure 2B). The current conductive member 250 is in the form of a member with the capability of reversible deformation along its longitudinal axis and thereby the exertion of a compressing force; said member 250 is preferably formed as a cylindrical spring. It is also preferred that the electrical conductive member 250 is made of titanium.

There is at least one current conducting member 255 in the electrical connector casing 235 arranged for providing electrical connection between the power supply and the electrode support 17 (see Figure 2B). The current conductive member 255 is in the form of a member with the capability of reversible deformation along its longitudinal axis and thereby the exertion of a compressing force; said member 255 is preferably formed as a cylindrical spring. It is also preferred that the electrical conductive member 255 is made of platinum. The use of electrical conductive members 250, 255 formed as resilient parts allows to eliminate the changes in dimension due to size deviations and temperature fluctuations.

The external walls of the half cells 210, 215, i.e. the walls not contacting with the electrode structure 10, are provided with a cathode side confining plate 220 and an anode side confining plate 225, respectively. The confining plates 220, 225 serve for protecting the half cells 210, 215 against the external mechanical impacts. Accordingly, the confining plates 220, 225 are made of a material with high mechanical strength, preferably of stainless steel. The water feeding connector 260, the hydrogen and water discharging connector 262 and the cathode side electrical connector casing 230 are firmly (but in a releasable manner) fixed into through-holes (not shown in the drawings) formed in the confining plate 220. Similarly, the ozone/oxygen gas discharging connector 265 and the anode side electrical connector casing 235 are firmly (but in a releasable manner) fixed into through-holes (not shown in the drawings) formed in the confining plate 225. Finally, in order to hold the cell 110' in one piece, to seal the electrode structure 10 constituting the central part of the cell 110' and to provide the required electrical and mechanical contacts between the parts of the cell 110' within the half cells 210, 215, through-bolts 285 are arranged in through-holes formed in the half cells 210, 215 and in the confining plates 220, 225 and said through-bolts 285 are fastened by screw nuts 290.

Regulation of the amount of ozone generated takes place by the magnitude of the direct current used for the electrolysis. The pressure of the gaseous ozone generated is preferably 1 to 50 bar, more preferably at most about 30 bar, before its feeding into the uniting element 120. Provision of the ozone source 110 as one or more electrolysis cells 110' makes the hazardous process of ozone handling highly safe. Further embodiments of the ozone source 110 applicable as part of the ozonolysis apparatus 100 according to the invention are disclosed in detail in International Publication Pamphlet No. WO2007/072098, entitled "*Ozone generating electrolysis cell*" filed on the same international filing date as that of the present patent application.

In its simplest form, the first reactor 130 constituting a first reaction section of the reactor unit is formed as a preferably tubular closed (preferentially cylindrical) member having a reaction channel 135 provided with an inlet and an outlet. The diffusion limited reaction of the sample solution and ozone, i.e. the ozonolysis reaction planned, takes place in the reactor 130, or rather within the reaction channel 135 thereof. The first reactor 130 is equipped with a primary temperature adjusting means 132 in heat transfer or heat exchange relation with the reaction channel 135. The temperature adjusting means 132 performs setting of a desired temperature of the sample solution containing the ozone bubbles. A preferred embodiment of the temperature adjusting means 132 is in the form of e.g. heating/cooling strands wound around a given section of the reaction channel 135 or a cascaded-type Peltier unit in contact with a given section of the reaction channel 135 through the wall thereof. The temperature adjusting means 132 is connected to the control unit 190 through an electric lead 136. The temperature adjusting means 132 is operated by the control unit 190 through the electric lead 136 on basis of the signal provided by a temperature sensor (not shown in the drawings) continuously measuring the temperature within the reactor 130. ,

In its simplest form, the second reactor 150 constituting a second reaction section of the reactor unit is formed as a preferably tubular closed (preferentially cylindrical) member having a reaction channel 155 provided with an inlet and an outlet. The chemical reaction of the intermediate/ozone/oxygen mixture leaving the reactor 130 and the additive added thereto in the uniting element 140, i.e. the decomposition/stabilization reaction of the intermediate, takes place in the reactor 150, or rather within the reaction channel 155 thereof. The second reactor 150 is equipped with a primary temperature adjusting means 152 in heat transfer or heat exchange relation with the reaction channel 155. The temperature adjusting means 132 performs setting of a desired temperature of the mixture of the intermediate and the additive. A preferred embodiment of the temperature adjusting means 152 is in the form of e.g. heating/cooling strands wound around a given section of the reaction channel 155 or a cascaded-type Peltier unit in contact with a given section of the reaction channel 155 through the wall thereof. The temperature adjusting means 152 is connected to the control unit 190 through an electric lead 156. The temperature adjusting means 152 is operated by the control unit 190 through the electric lead 156 on basis of the signal provided by a temperature sensor (not shown in the drawings) continuously measuring the temperature within the reactor 150. Temperatures within the reaction channels 135, 155 of the reactors 130, 150, respectively, can be controlled independently of one another by means of the control unit 190.

In embodiments of the first 130 and second 150 reactors formed as simple tubes the inner diameters of the reaction channels 135, 155 are preferably at most 4 to 5 mm, while the lengths of the reaction channels 135, 155 are generally 30 to 100 mm, more preferably 40 to 50 mm. In general, the lengths of the reaction channels 135, 155 are preferably chosen to enable the accomplishment of the reactions planned in the reaction mixture entered through the inlet while said mixture passes through the reaction channel.

The first reactor 130 is connected into between suitably formed sections of the pipes 106 and 107 in a removable manner, preferably by a detachable connection. Similarly, the second reactor 150 is connected into between suitably formed sections of the pipes 107 and 108 in a removable manner, preferably by a detachable connection, too. Accordingly, in a possible embodiment of the reactors 130, 150 the inlets and the outlets of the reaction channels 135, 155 are threaded and connected into between the pipes 106 and 107 and/or the pipes 107 and 108, respectively, by flare joints with making use of appropriate sealings.

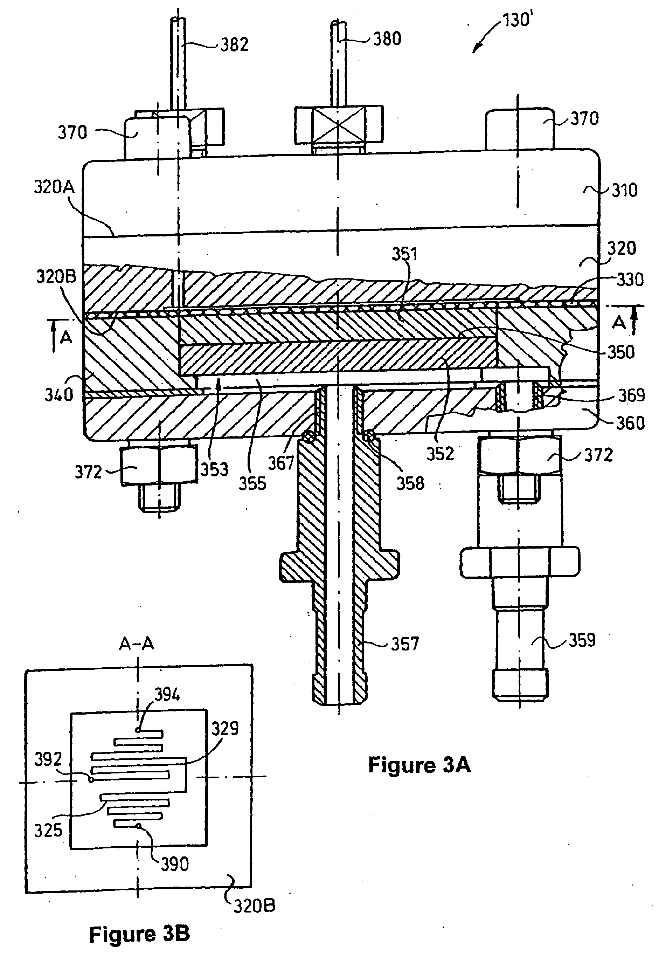

Figures 3A and 3B illustrate a further possible embodiment of the first 130 and second 150 reactors used in the ozonolysis apparatus 100 in the form of a microfluidic reactor 130'. Said microfluidic reactor 130' is of a particular construction and can be manufactured with low costs.

In the prior art, the term "microfluidic reactor" commonly refers to a sealed channel provided with an inlet and an outlet and used to accommodate a reaction mixture flowing continuously or intermittently with short periods of temporary stops, wherein the dimension of said channel perpendicular to the direction of flow of the reaction mixture does not exceed 0.5 mm. The microfluidic reactor 130' illustrated in Figure 3A comprises a reactor sheet 320 defined by a first face 320A and a second face 320B, a first limiting member 310 in contact with the first face 320A of the reactor sheet 320, a closing member 330 covering the second face 320B of the reactor sheet 320, a supporting member 340 in contact with the closing member 330 along its peripheries and a second limiting member 360 abutting on the face of the supporting member 340 opposite to the closing member 330. The closing member 330, the supporting member 340 and the limiting member 360 together define a cooling chamber 353 therebetween. Within the cooling chamber 353, a temperature control unit 350 is arranged in contact with the closing member 330. The temperature control unit 350 is firmly fixed in its place by the supporting member 340. In this arrangement, the temperature control unit 350 is separated from the second limiting member 360 by a clearance, that is, the temperature control unit 350 and the second limiting member 360 do not contact each other. The cooling chamber 353 is in communication with the external environment through preferably threaded through-holes 367, 369 formed in the second limiting member 360.

The first and second limiting members 310, 360 serves for holding together the elements arranged therebetween and protecting them against external mechanical impacts. Accordingly, the limiting members 310, 360 are made of steel, for example stainless steel, of high mechanical strength. The limiting member 310 is further provided with through-holes (not shown) to allow communication between the external environment and the channel formed in the reactor sheet 320. One of the through-holes 367, 369 receives a coolant feeding means 357 opening into a flow channel 355 defined by the clearance adjacent to the temperature control unit 350 of the reactor 130' in a sealed and releasable manner. The sealed connection of the coolant feeding means 357 is provided by a gasket 358 arranged between the limiting member 360 and the coolant feeding means 357 itself. The gasket 358 is preferably formed as an O-type ring. The other one of the threaded through-holes 367, 369 formed in the limiting member 360 is adapted to receive a coolant discharging means 359 in a sealed and releasable manner, wherein said coolant discharging means 359 opens from the clearance 355. The sealed connection of the coolant discharging means 359 is provided by a gasket (not shown in the figures) arranged between the limiting member 360 and the coolant discharging means 359 itself, wherein the gasket is preferably formed as an O-type ring.

The reactor sheet 320 is made of a chemically resistant, easily machinable heat-resistant material. The material of the reactor sheet 320 is a chemically resistant plastic material, preferably a fluorinated and/or chlorinated polymer, more preferably PTFE. The reaction channel 325, that is used to ensure a proper space for the reactants or the mixture thereof during the operation of the reactor 130', as well as to accommodate the chemical reaction planned (i.e. the ozonolysis reaction or the reaction for decomposing/stabilizing the intermediate), is formed in the face 320B of the reactor sheet 320; details of the reaction channel 325 are shown in Figure 3B.

As it can be seen from Figure 3B the reaction channel 325 is provided with pass-throughs 390, 392, 394 in flow communication with connectors 380, 382, 384, respectively, mounted into through-holes of the limiting member 310. The pass-throughs 390, 392 are used to feed the reactants, whereas the pass-through 394 is used to discharge the reaction product of the chemical reaction taking place in the reaction mixture produced by an uniting element 329 having an Y-shape and serving as a mixer integrated into the reaction channel 325. The length of a section of the reaction channel 325 extending between the uniting element 329 and the pass-through 394 is chosen in such a way that the desired chemical reaction of the reactants getting into contact with one another due to the uniting element 329 be performed before the reactants reach the pass-through 394. As shown in Figure 3B, in order to facilitate the design of the reactor 130' with reduced dimensions, the reaction channel 325 consists of relatively long sections running in parallel and relatively short sections running perpendicularly to the former ones. Another advantage of this topology of the reaction channel 325 is that the temperature of the reactants/reaction mixture can be changed quickly and reliably due to the large ratio of the channel surface to the channel volume.

The reaction channel 325 is formed in the face 320B in such a manner that after properly clamping the reactor sheet 320, a machining tool with a rolling machining surface - preferably a ball-type machining tool - is pressed onto the face 320B by applying a compressive force chosen depending on the material and the plasticity of the reactor sheet 320, wherein said rolling machining surface is guided, according to a program, in a guiding (or controlling) device put onto the place of the limiting member 310. The value of the applied compressive force is chosen to cause the ball of the machining tool to intrude into the body of the reactor sheet 320 to an extent of the entire desired depth of the reaction channel 325 to be formed. The reaction channel 325 is then formed by continuously advancing the ball-type machining tool along a predetermined path of the required reaction channel 325 according to an appropriate adjustment of the control, wherein the rate of advance is typically in the range of 0.1 to 5 mm/sec. During the continuous advancing motion of the machining tool with the rolling machining surface, the material of the reactor sheet 320 is getting dense along the peripheries of the reaction channel 325 and due to the shearing forces arising becomes squeezed out, which results in the formation of a sealing edge along each of the opposite peripheries of the reaction channel 325. Dimensions of the reaction channel 325 and the sealing edges depend on the diameter of the machining surface of the machining tool. In particular, if said ball-type machining tool is used, the width of the reaction channel 325 and the size of the sealing edges formed together with the reaction channel 325 can be adjusted by changing the diameter of the ball of the machining tool. The depth of the reaction channel 325 may be adjusted by changing the height position of the ball of the ball-type machining tool.

Sealing of the reaction channel 325 formed in the reactor sheet 320 is carried out by placing the closing member 330 onto the face 320B or the sealing edges projecting therefrom, and by applying a compressive force perpendicular to the plane of the closing member 330. Due to the applied force, the sealing edges become deformed that results in a sealed joint between the reactor sheet 320 and the closing member 330. It should be noted that in this case, the compressive force is distributed only over the sealing edges of the face 320B, instead of the whole face 320B, which yields more reliable sealing. For the closing member 330, a sheet-like member/film made of a material with good thermal conductivity and chemical resistance, as well as having smooth surfaces is used. If good thermal conductivity is an essential requirement, the closing member 330 is made of a PTFE film with a thickness of up to 20 µm.

After sealing the reaction channel 325, the reactor sheet 320 is provided with through-holes in the full thickness of the reactor sheet 320 at the pass-throughs 390, 392, 394 in order to allow the communication between the connectors mounted into the limiting member 310 and the pass-throughs 390, 392, 394.

It is preferred that the supporting member 340 is in the form of a square frame made of aluminum.

The temperature control unit 350 accommodated within the supporting member 340 is arranged at an opposite surface of the closing member 330 relative to the reaction channel 325, in a position where it is in contact with the closing member 330, as shown in Figure 3A. A preferred embodiment of the temperature control unit 350 is preferably formed as a cascaded-type Peltier-unit (comprising a plurality of, but preferably two Peltier-elements in contact with one another), the operation of which is based on the Peltier-effect. As it is obvious for one skilled in the art, a Peltier-element is a device comprising two thin ceramic sheets and a plurality of semiconductor sheets therebetween, which provides a constant difference of temperature between its two sides when an appropriate current and voltage is applied on the device. In its simplest form, there are two different metal layers on the bottom side of the Peltier-element through which a current is flowing which results in a thermal flow between the metal layers. Thus a cold side and a warm side are obtained. The cold side of a first Peltier-element 351 of the temperature control unit 350 used in the microfluidic reactor 130' is in contact, through the closing member 330, with the reactor sheet 320 or the reactants/reaction mixture accommodated within the reaction channel 325. At the same time, the warm side of the first Peltier-element 351 of the temperature control unit 350 used in the microfluidic reactor 130' is in contact with the cold side of a second Peltier-element 352. The temperature control unit 350 used to control the temperature of the reactor 130' is formed by an integrated unit of the two aforementioned Peltier-elements 351, 352, preferably within a common casing.

The first limiting member 310, the reactor sheet 320, the closing member 330 and the supporting member 340 of the temperature control unit 350 of the microfluidic reactor 130' are held and clamped together by an appropriate fastening mechanism in order to create and maintain a perfect seal between the reactor sheet 320 and the closing member 330. The fastening mechanism is preferably formed by through-bolts 370 inserted into through-holes (not shown) formed in each one of said parts and crossing over the entire thickness of the reactor 130', and by nuts 372 screwed thereon.

In the microfluidic reactor 130' applicable within the ozonolysis apparatus 100 according to the invention, the entire surface used to form the fluidic construction is 25 cm², the channel has a length of 65 mm, the average diameter of the channel is 400 µm, the depth of the channel is also 400 µm, the number of inlets and outlets is three, a single uniting element is used, the lowest reachable temperature within the channel is -50°C (at a thermal load of +5 W), the highest reachable temperature within the channel is 350°C, the maximum operational pressure of the reactor is 30 bar, and the maximum rate of change in temperature is 8°C/sec (within the range of 0 to 20°C).

Possible further embodiments of the reactors 130, 150 of the ozonolysis apparatus 100 according to the invention are disclosed in detail in International Publication Pamphlet No. WO2007/072099 , entitled "*Method of forming a sealed channel of a microfluidic reactor and a microfluidic reactor comprising such channel"* filed on the same international filing date as that of the present patent application. It should be noted that in case of the microfluidic reactor 130' shown in Figures 3A and 3B is used as the reactors 130, 150, the uniting elements 120, 140 are integrated into the reaction channel 325 formed in the microfluidic reactor 130' and hence there is no need to use such further elements. In such a case, the outlet of the feed pump 102 and the outlet of the dispensing valve 112 are connected directly to the inlets of the microfluidic reactor 130' forming the first reactor 130, whereas the outlet of the feed pump 172 and the outlet of the reactor 130 are connected directly to the inlets of the microfluidic reactor 130' forming the second reactor 150. Moreover, when using said microfluidic reactor 130', the temperature adjusting means 132,152 are provided by the microfluidic reactor's 130' own temperature control unit 350.

A possible yet further embodiment of the second reactor 150 can be provided in the form of a packed column with a reactive charge. In such a case, the reactive charge preferably contains the additive required for the decomposition/stabilization reaction of the intermediate in solid phase, and hence there is no need to supply a liquid phase additive. As such packed columns, e.g. the replaceable cartridge reactor a diselosed in International Publication Pamphlet No. WO2006/021822 can be preferentially used.

The pressure adjusting means 160 is an electronically controlled, motor driven precision pressure adjusting valve that is arranged at the end of the flow path and adjusts the pressure needed for the ozonolysis reaction and maintains it at a constant value within the flow path. The pressure adjusting valve, in consequence of its construction, is highly sensitive and is capable of setting the value of the pressure in extremely tiny steps, i.e. almost continuously. The operational range of the pressure adjusting means 160 ranges from 1 bar to 100 bar, preferably from 10 bar to 30 bar. The pressure adjusting means 160 is actuated by the control unit 190 by means of appropriate trigger signals sent via the electrical lead 197.

The pipes 106, 107, 108, 114, 176 are formed as capillaries with inner diameters falling within the range of 0.05 to 5 mm, preferably of 0.05 mm and made of a pressureproof and chemically resistant material.

As it is shown in Figure 1, a possible further embodiment of the ozonolysis apparatus 100 according to the invention is also equipped with a secondary temperature adjusting means 185. The secondary temperature adjusting means 185 is in heat transfer relation with the primary temperature adjusting means 132 of the first reactor 130 and/or the primary temperature adjusting means 152 of the second reactor 150 via heat exchangers 186 and 187, respectively. (When a microfluidic reactor 130' shown in Figure 3A is used as each of the reactors 130, 150, the heat exchangers 186, 187 are in heat transfer relation with the reactors' own temperature control units 350.) On the other part, the secondary temperature adjusting means 185 is in heat transfer/heat exchange relation with its external environment. The secondary temperature adjusting means 185 is operated by the control unit 190 by means of appropriate trigger signals sent via an electrical lead 189.

In what follows, operation of the laboratory scale ozonolysis apparatus 100 is discussed in detail.

After starting the ozonolysis apparatus 100, as a response to a signal from the control unit 190, the feed pump 102 commences to feed the sample solution from the liquid reservoir 104 through the pipe 106 into the flow path at a previously set constant flow rate falling preferably between 0.1 ml/min and 10 ml/min, and tipically being 0.25 ml/min. Simultaneously, as a response to a first signal from the control unit 190, the dispensing valve 112 opens, then as a response to a second signal following the first one within a relatively short period of time it closes and then repeats this opening-closing operation until it receives a different command from the control unit 190. As a consequence of the opening-closing operation of the dispensing valve 112, doses of given amounts of gaseous ozone are delivered one after the other from the ozone source 110 through the pipe 114 into the flow path. To maintain feeding of ozone, the pressure of the gaseous ozone flowing through the pipe 114 will be all the time higher than the pressure of the sample solution flowing through the pipe 106. Simultaneously with the very first opening of the dispensing valve 112, the pressure adjusting means 160 - as a response again to a signal from the control unit 190 - closes to build up a pressure prescribed in the flow path (and required by the ozonolysis reaction). The sample solution supplied and the gaseous ozone meet in the uniting element 120 wherein they get mixed. The sample solution containing ozone bubbles flows via the outlet of the uniting element 120 into the first reactor 130 through the pipe 106. Within the reaction channel 135 of the reactor 130, temperature of the sample solution containing ozone bubbles is set to the desired value by the temperature adjusting means 132 under the supervision of the control unit 190. Meanwhile, the sample solution fed into the reactor 130 progresses towards the outlet of the reactor 130 in the reaction channel 135. During the progression, the desired ozonolysis reaction takes place within the reaction channel 135. The extension of reaction to the whole sample solution within the reaction channel 135 is assured on the one part by adjusting/maintaining the temperature within the reaction channel 135 and on the other part by means of the pressure maintained in the flow path by the pressure adjusting means 160. The intermediate that is produced from the sample solution passing through the reaction channel 135, optionally mixed with gaseous ozone and oxygen, flows through the output of the reactor 130 and the pipe 107 into the uniting element 140. Simultaneously with this, as a response to the signal from the control unit 190, the solution of additive is fed at a preset flow rate from the liquid reservoir 174 to the other inlet of the uniting element 140 by the feed pump 172. Within the uniting element 140, the intermediate mixes with the solution of additive. From the uniting element 140, the mixture of the intermediate and the solution of additive enters the second reactor 150, within the reaction channel 155 of which the decomposition/stabilization reaction of the intermediate takes place at a temperature set by the temperature adjusting means 152 under the supervision of the control unit 190. The mixture of the final product, ozone and oxygen leaving the outlet of the reactor 150 enters the product receptacle 180 through the pressure adjusting means 160. By means of suitable units, hazardous ozone is extracted from the mixture being discharged into the product receptacle 180 and is either destructed by e.g. heating or via catalytic processes or recirculated to the outlet of the ozone source 110. Meanwhile, the oxygen content of the mixture simply escapes to the environment.

Besides the structural elements required for accomplishing the ozonolysis reaction, the ozonolysis apparatus 100 according to the invention is also equipped with a display unit (not illustrated in the drawings) and with a keyboard for data input. Operation of the ozonolysis apparatus 100 according to the invention and of the units thereof is assured by a direct current power supply not shown in the drawings.

The total volume of the continuously connected flow path of the flow-type laboratory scale ozonolysis apparatus 100 according to the invention is at most 50 cm³, more preferably at most 25 cm³.

It is apparent for a person skilled in the relevant art that a great number of modifications of the ozonolysis apparatus 100 are possible, and furthermore the apparatus itself can be equipped with further additional units. In particular, one of the modifications relates to the ozone source 110; the ozone source 110 can be also implemented e.g. with a unit measuring the concentration of the gaseous ozone generated by the ozone source 110 itself and/or with a drying unit decreasing the water content of the gaseous ozone generated by the ozone source 110 itself. The drying unit might preferably be a freezing-out type drying unit.

In what follows, the ozonolysis apparatus 100 according to the invention, as well as its application and advantages will be illustrated by examples. The ozonolysis apparatus 100 according to the invention is especially suitable for accomplishing the reactions of the examples. The reason for this is that in case of reactions taking place in the heterogeneous phase (gas/liquid) the length of the vital contacting period and the size of the contacting surface can be controlled. In addition to this, most of the ozonolysis reactions require the application of temperatures below the room temperature, and in most of the reactions the further chemical process of the ozonides also requires a cooled environment. As exothermic reactions take place here, it is essential to divert the heat of reaction rapidly and in a reliable manner which is facilitated to a great extent by the temperature adjusting system used in the ozonolysis apparatus 100 according to the invention. Since in case of an ozonolysis apparatus 100 according to the invention a well-defined period of time elapses between the chemical reaction for producing the ozonide and the chemical reaction for decomposing it (as the mixture leaving the first reactor 130 should reach the uniting element 140), the appearance of side reactions can be decreased to a great extent, i.e. the target compound can be produced at much higher yields.

### EXAMPLE 1

A solution (1:1) of 5-metil-1-H-indol in methanol/dichloromethane with a concentration of 0.025 mol/l is fed into the reaction channel 135 of a first reactor 130 provided as the microfluidic reactor by means of the feed pump 102 from the liquid reservoir 104 of the ozonolysis apparatus 100. Furthermore, a gaseous mixture of ozone and oxygen with a constant amount of 5% volume by volume ozone is fed into the reaction channel 135 of the first reactor 130 provided as the microfluidic reactor illustrated in Figure 3A by means of a controlled actuation of the dispensing valve 112. Said reactants can mix within the reaction channel 135. The flow rate of the solution of the 5-metil-1-H-indol through the reactor 130 is kept constant at 0.25 ml/min, wherein a pressure of 5 bar is.continuously maintained within the reactor 130 during the entire process. The reaction is performed at the temperature of 0°C. The mixture with an ozonide content led out from the reactor 130 is directed into the reaction channel 155 of a second reactor 150 through the pipe 107. Here the second reactor 150 is formed e.g. as a tubular reactor providing a reaction channel 155 with an inlet and an outlet and containing sodium borohydride (NaBH₄) in an immobilized form. The reaction is performed at the temperature of -15°C. The second reagent NaBH₄ ensures the decomposition of the intermediate of the reaction (the ozonide produced). The average residence time within the reactor 150 is in the order of seconds. The reaction product is N-(2-hydroxy methyl-4-methyl-phenyl)-formamide. According to a high-pressure liquid chromatography (HPLC) analysis, the degree of purity of the reaction product is 98%.

The yield of reactions performed by conventional reactors is about 60 to 65%, which primarily comes from the difficultly controllable reaction conditions and thus from the proportion of the side products of the reaction [see for example the Journal of the Chemical Society (1953), pp. 3440-3443 or the Journal of the Chemical Society (1950), pp. 612-618]. In case of a reaction accomplished under improved conditions, the yield decreases in proportional to an increase in the degree of purity.

### EXAMPLE 2

Our study of continuous flow ozonolysis with an ozonolysis apparatus 100 according to the invention involves two major groups of compounds: aromatic hydrocarbons and indoles. The ozonolysis of these species via "classical" methods and the results thereof are well-known. After the ozonolysis, a reductive workup is chosen to quench the intermediates. For this, a reactor 150 filled with sodium borohydride proved to be the best choice. The results obtained under the applied conditions show that neither the reaction rate nor the level of selectivity of the ozonolysis performed by the ozonolysis apparatus 100 according to the invention depends on temperature. This conclusion is supported by the experiments carried out both for stilbene and indole: the distribution of products is the same at the temperatures of -40°C, -20°C, 0°C and 20°C. Using excess olefin over the ozone, the extent of conversion remains the same at all temperatures applied (Table 1). This invariability of conversion at various temperatures indicates that no considerable secondary or side reaction takes place with a rate of the same order of magnitude as that of the ozonolysis reaction within the ozonolysis apparatus 100 according to the invention.

**Table 1: Conversions of indole measured during ozonolysis reactions thereof performed at different temperatures (c=0,05M).**

| T (reactor, °C) | conversion (%) |
|---|---|
| -60 | 20 |
| -40 | 60 |
| -20 | 60 |
| 0 | 60 |
| 20 | 60 |

According to the literature of ozonolysis reactions, to avoid undesired side reactions, vast majority of such type of reactions should be performed at low temperatures. In light of this, it was unexpected in our studies carried out by making use of the ozonolysis apparatus according to the invention that the extent of conversion is independent of the temperature within the reactor. This fortuitous finding clearly means that various ozonolysis reactions can be performed preferably even at ambient temperature by means of the ozonolysis apparatus according to the invention without a decrease in the extent of conversion.

In particular, the ozonolysis of stilbene and tetraphenyl-ethylene within the ozonolysis apparatus 100 according to the invention at ambient temperature results in benzylalcohol and diphenyl carbinol, respectively, as main products. The extents of conversion for said reactions are collected in Table 2.

**Table 2**

| reactant / olefin | conversion at ambient temperature (%) |
|---|---|
| stilbene | 90 |
| tetraphenyl-ethylene | 90 |

The results of the above discussed experiments prove that the ozonolysis apparatus according to the present invention - contrary to the apparatuses for performing ozonolysis reactions which belong to the prior art and are used nowadays - has an excellent heat transfer coefficient. As the consequence of its excellent heat-removal capability, the ozonolysis apparatus according to the invention thus assures the removal of heat generated within the reactor during the reaction at a proper rate even in case of exothermic reactions accompanied by intensive generation of heat, and thereby it inhibits the overheating of the reaction mixture and hence the start and course of undesired side reactions. On the contrary, traditional reactors are unable to provide heat-removal of sufficent rapidity, and hence to avoid side reactions when using a traditional reactor an overcooling and then a slow heating of the reaction mixture is required. This leads to an increase in the time period needed for the target reaction to take place, and furthermore it undesirably decreases the rate of reaction.

## Claims

1. A flow-type laboratory scale ozonolysis apparatus for performing ozonolysis reaction of a given substance, the apparatus (100) comprising a liquid reservoir (104), a feed pump (102) in the form of a liquid pump generating a constant volume rate, a uniting element (120) with two inlets and an outlet, a reactor unit and a pressure-adjusting means (160), all connected into a flow path, the apparatus (100) further comprising an ozone source (110) and a dispensing valve (112) transmitting a gas stream only in a single direction and being installed between the ozone source (110) and one of the inlets of the uniting element (120), wherein the pressure-adjusting means (160) is arranged after the reactor unit and is provided with an electrically govemed control, ***characterized in* that** the liquid reservoir (104) contains at least the substance, as a solute, to be subjected to the ozonolysis reaction and the reactor unit consists of first and second reactor zones being different as to their function, wherein the outlet of the first reactor zone is connected to the inlet of the second reactor zone in the flow path and a substance intake is inserted into the flow path between the reactor zones, and wherein the ozone source (110) is provided as an ozone source generating ozone in-situ by electrolysis and wherein the total inner volume measured along the flow path between the feed pump (102) and the pressure adjusting means (160) is at most 50 cm³.

2. The laboratory scale ozonolysis apparatus (100) of claim 1, ***characterized in* that** the reactor zones of the reactor unit are provided as physically separate first and second reactors (130, 150).

3. The laboratory scale ozonolysis apparatus (100) of claim 2, ***characterized in* that** the first reactor (130) is provided with a temperature adjusting means (132) in heat transfer relation with the reactor (130).

4. the laboratory scale ozonolysis- apparatus (100) of claim 2, ***characterized in* that** the second reactor (150) is provided with a temperature adjusting means (152) in heat transfer relation with the reactor (150).

5. The laboratory scale ozonolysis apparatus (100) of claim 2, ***characterized in* that** the substance intake is formed as a uniting element (140) with two inlets and an outlet, wherein the outlet of the first reactor (130) is connected to one of the inlets of the uniting element (140), the inlet of the second reactor (150) is connected to the outlet of the uniting element (140) and a second liquid reservoir (174) is connected to the other inlet of the uniting element (140) through a second feed pump (172), wherein the second liquid reservoir (174) contains at least an additive needed for the completion of ozonolysis reaction.

6. The laboratory scale ozonolysis apparatus (100) of claim 5, ***characterized in* that** the first reactor (130) is provided as a microfluidic reactor (130) having an inlet, an outlet and a reaction channel (325) for accommodating chemical reactions, the microfluidic reactor (130') comprising a sealed reaction channel (325) prepared in the surface of a reactor sheet (320) by cold forming and sealed by a closing member (330) pressed onto said surface of the reactor sheet (320) and a temperature control unit (350) arranged in contact with a surface of the closing member (330) opposite to the reaction channel (325) and being in heat transfer relation with the reaction channel (325) through the closing member (330).

7. The laboratory scale ozonolysis apparatus (100) of claim 6, ***characterized in* that** the uniting element (120) is formed as an integral member of the reaction channel (325).

8. The laboratory scale ozonolysis apparatus (100) of claim 6, ***characterized in* that** the temperature adjusting means (132) is provided by the temperature control unit (350).

9. The laboratory scale ozonolysis apparatus (100) of claim 2, ***characterized in* that** the second reactor (150) is provided as a microfluidic reactor (130') with the same construction as that of the first reactor (130).

10. The laboratory scale ozonolysis apparatus (100) of claim 2, ***characterized in* that** the second reactor (150) is provided as a packed column with a reactive charge, wherein the charge contains an additive needed for the completion of ozonolysis reaction.

11. The laboratory scale ozonolysis apparatus (100) of claim 1, ***characterized in* that** the ozone source (110) is provided as at least one asymmetric pressure ozone generating electrolysis cell (110').

12. The laboratory scale ozonolysis apparatus (100) of claim 11, ***characterized in* that** the ozone generating electrolysis cell (110') comprises
- a cathode (13);
- an anode (16) comprising a mixture of lead(IV) oxide and polytetrafluoroethylene;
- a membrane (15) arranged between the cathode (13) and the anode (16); and
- an electrically conducting, liquid and gas permeable first electrode support (17) in contact with a side of the anode (16) located opposite to the membrane (15), said side of the electrode support (17) having a surface covered with a platinum-containing layer, wherein the material of the anode (16) is a mixture prepared by high-pressure moulding of lead(IV) oxide.grains of colloid size and polytetrafluoroethylene filaments having a dimension of at most 1 mm.

13. The laboratory scale ozonolysis apparatus (100) of claim 1, ***characterized in* that** it further comprises a central control unit (190) which is connected to the dispensing valve (112), the feed pump (102) and the pressure adjusting means (160) through appropriate electrical connections.

14. The laboratory scale ozonolysis apparatus (100) of claims 3 and 13, ***characterized in* that** the temperature adjusting means (132) of the first reactor (130) is connected with the central control unit (190) through an appropriate electrical connection.

15. The laboratory scale ozonolysis apparatus (100) of claims 4 and 13, ***characterized in* that** the temperature adjusting means (152) of the second reactor (150) is connected with the central control unit (190) through an appropriate electrical connection.

16. The laboratory scale ozonolysis apparatus (100) of claims 14 and 15, ***characterized in* that** it further comprises a secondary temperature adjusting means (185) which is in heat exchange relation with the temperature adjusting means (132) of the first reactor (130) and/or the temperature adjusting means (152) of the second reactor (150), and is connected with the central control unit (190) through an appropriate electrical connection.

17. The laboratory scale ozonolysis apparatus (100) of claim 1, ***characterized in* that** the uniting element (120) is provided with an end sheet at the inlet thereof through which ozone is fed into the flow path, said end plate having perforations to decrease the size of the ozone bubbles to be introduced.

18. A laboratory scale method of performing ozonolysis reaction of a substance solved in a solvent, comprising the steps of
(i) supplying a given amount of substance being solved to be subjected to ozonolysis reaction into a flow path by means of a feed pump (102) at a substantially constant volume rate;
(ii) feeding ozone by a dispensing valve (112) into the flow path in a section located after the substance supplying position.in the form of microbubbles, said ozone being produced in-situ by electrolysis;
(iii) leading solved substance through a reactor unit comprising first and second reactor zones arranged in a section of the flow path located after the ozone feeding position;
(iv) supplying an additive needed for the completion of ozonolysis reaction into the flow path after the first reactor zone of the reactor unit;
(v) maintaining the pressure of the reaction in a given pressure range by means of a pressure adjusting means (160) arranged in the flow path after the reactor unit;
(vi) collecting the product generated in the second reactor zone of the reactor unit in a product receptacle (180) connected to the end of the flow path, wherein the total inner volume measured along said flow path, also including the volume of the reactor unit, is at most 50 cm³.

19. The laboratory scale method of performing ozonolysis reaction according to claim 18, ***characterized in* that** the ozone is supplied into the flow path intermittently..

20. The laboratory scale method of performing ozonolysis reaction according to claim 18, ***characterized in* that** the temperature of the solved substance is changed to a prescribed temperature of the reaction to be performed in the first reactor zone of the reactor unit.

21. The laboratory scale method of performing ozonolysis reaction according to claim 20, ***characterized in* that** a reaction resulting in decomposition or stabilization of the intermediate produced by the reaction performed in the first reactor zone is carried out in the second reactor zone of the reactor unit.

22. The laboratory scale method of performing ozonolysis reaction according to claim 21, *charticierized in* that the temperature or the intermediate produced in the first reactor zone and the temperature of the additive supplied into the flow path are changed in the second reactor zone of the reactor unit to a prescribed temperature of the reaction to be performed in the second reactor zone of the reactor unit.

## Patentansprüche

1. Labormaßstab-Ozonolyseapparatur vom Strömungstyp zur Durchführung einer Ozonolysereaktion einer bestimmten Substanz, wobei die Apparatur (100) einen Flüssigkeitsbehälter (104), eine Förderpumpe (102) in Form einer einen gleichmäßigen Volumendurchsatz erzeugenden Flüssigkeitspumpe, ein Vereinigungselement (120) mit zwei Einlässen und einem Auslass, eine Reaktoreinheit und ein Druckeinstellmittel (160) alle in einem Strömungsweg verbunden umfasst und wobei die Apparatur (100) ferner eine Ozonquelle (110) und ein Abgabeventil (112), das einen Gasstrom nur in einer einzigen Richtung überträgt und zwischen der Ozonquelle (110) und einem der Einlässe des Vereinigungselements (120) eingebaut ist, umfasst, wobei das Druckeinstellmittel (160) hinter der Reaktoreinheit angeordnet ist und mit einer elektrisch geregelten Steuerung versehen ist, **dadurch gekennzeichnet, dass** der Flüssigkeitsbehälter (104) zumindest die der Ozonolysereaktion zu unterziehende Substanz als gelösten Stoff enthält und die Reaktoreinheit aus einer ersten und einer zweiten Reaktorzone besteht, die in Bezug auf ihre Funktion verschieden sind, wobei der Auslass der ersten Reaktorzone mit dem Einlass der zweiten Reaktorzone im Strömungsweg verbunden ist und ein Substanzzulauf in den Strömungsweg zwischen den Reaktorzonen eingebracht ist und wobei die Ozonquelle (110) als eine Ozon in situ durch Elektrolyse erzeugende Ozonquelle vorgesehen ist und wobei das gesamte Innenvolumen gemessen entlang des Strömungswegs zwischen der Förderpumpe (102) und dem Druckeinstellmittel (160) höchstens 50 cm³ beträgt.

2. Labormaßstab-Ozonolyseapparatur (100) nach Anspruch 1, **dadurch gekennzeichnet, dass** die Reaktorzonen der Reaktoreinheit als physikalisch getrennter erster und zweiter Reaktor (130, 150) vorgesehen sind.

3. Labormaßstab-Ozonolyseapparatur (100) nach Anspruch 2, **dadurch gekennzeichnet, dass** der erste Reaktor (130) mit einem Temperatureinstellmittel (132) in Wärmeübertragungsbeziehung mit dem Reaktor (130) versehen ist.

4. Labormaßstab-Ozonolyseapparatur (100) nach Anspruch 2, **dadurch gekennzeichnet, dass** der zweite Reaktor (150) mit einem Temperatureinstellmittel (152) in Wärmeübertragungsbeziehung mit dem Reaktor (150) versehen ist.

5. Labormaßstab-Ozonolyseapparatur (100) nach Anspruch 2, **dadurch gekennzeichnet, dass** der Substanzzulauf als Vereinigungselement (140) mit zwei Einlässen und einem Auslass ausgebildet ist, wobei der Auslass des ersten Reaktors (130) mit einem der Einlässe des Vereinigungselements (140) verbunden ist, der Einlass des zweiten Reaktors (150) mit dem Auslass des Vereinigungselements (140) verbunden ist und ein zweiter Flüssigkeitsbehälter (174) durch eine zweite Förderpumpe (172) mit dem anderen Einlass des Vereinigungselements (140) verbunden ist, wobei der zweite Flüssigkeitsbehälter (174) mindestens ein Additiv enthält, das für die Vollendung der Ozonolysereaktion benötigt wird.

6. Labormaßstab-Ozonolyseapparatur (100) nach Anspruch 5, **dadurch gekennzeichnet, dass** der erste Reaktor (130) als mikrofluidischer Reaktor (130') mit einem Einlass, einem Auslass und einem Reaktionskanal (325) zur Aufnahme chemischer Reaktionen vorgesehen ist, wobei der mikrofluidische Reaktor (130') Folgendes umfasst: einen verschlossenen Reaktionskanal (325), der in der Oberfläche einer Reaktorplatte (320) durch Kaltformen hergestellt und durch ein auf die Oberfläche der Reaktorplatte (320) gepresstes Verschlusselement (330) verschlossen ist, und eine Temperaturregeleinheit (350), die in Kontakt mit einer Oberfläche des Verschlusselements (330) gegenüber dem Reaktionskanal (325) angeordnet ist und durch das Verschlusselement (330) in Wärmeübertragungsbeziehung mit dem Reaktionskanal (325) steht.

7. Labormaßstab-Ozonolyseapparatur (100) nach Anspruch 6, **dadurch gekennzeichnet, dass** das Vereinigungselement (120) als einstückiges Element des Reaktionskanals (325) ausgebildet ist.

8. Labormaßstab-Ozonolyseapparatur (100) nach Anspruch 6, **dadurch gekennzeichnet, dass** das Temperatureinstellmittel (132) durch die Temperaturregeleinheit (350) bereitgestellt ist.

9. Labormaßstab-Ozonolyseapparatur (100) nach Anspruch 2, **dadurch gekennzeichnet, dass** der zweite Reaktor (150) als mikrofluidischer Reaktor (130') mit der gleichen Konstruktion wie derjenigen des ersten Reaktors (130) vorgesehen ist.

10. Labormaßstab-Ozonolyseapparatur (100) nach Anspruch 2, **dadurch gekennzeichnet, dass** der zweite Reaktor (150) als Füllkörpersäule mit einer reaktiven Packung vorgesehen ist, wobei die Packung ein Additiv enthält, das für die Vollendung der Ozonolysereaktion benötigt wird.

11. Labormaßstab-Ozonolyseapparatur (100) nach Anspruch 1, **dadurch gekennzeichnet, dass** die Ozonquelle (110) als mindestens eine Ozon erzeugende Elektrolysezelle (110') mit asymmetrischem Druck vorgesehen ist.

12. Labormaßstab-Ozonolyseapparatur (100) nach Anspruch 11, **dadurch gekennzeichnet, dass** die Ozon erzeugende Elektrolysezelle (110') Folgendes umfasst:
- eine Katode (13);
- eine Anode (16), die eine Mischung aus Blei(IV)-oxid und Polytetrafluorethylen umfasst;
- eine Membran (15), die zwischen der Katode (13) und der Anode (16) angeordnet ist; und
- einen elektrisch leitenden, flüssigkeits- und gasdurchlässigen ersten Elektrodenträger (17) in Kontakt mit einer gegenüber der Membran (15) angeordneten Seite der Anode (16), wobei die Seite des Elektrodenträgers (17) eine mit einer platinhaltigen Schicht bedeckte Oberfläche aufweist, wobei das Material der Anode (16) eine durch Hochdruckformung hergestellte Mischung aus Blei(IV)-oxid-Teilchen in Kolloidgröße und Polytetrafluorethylen-Fasern mit einer Abmessung von höchstens 1 mm ist.

13. Labormaßstab-Ozonolyseapparatur (100) nach Anspruch 1, **dadurch gekennzeichnet, dass** sie ferner eine zentrale Steuereinheit (190) umfasst, welche durch entsprechende elektrische Anschlüsse mit dem Abgabeventil (112), der Förderpumpe (102) und dem Druckeinstellmittel (160) verbunden ist.

14. Labormaßstab-Ozonolyseapparatur (100) nach Anspruch 3 und 13, **dadurch gekennzeichnet, dass** das Temperatureinstellmittel (132) des ersten Reaktors (130) durch einen entsprechenden elektrischen Anschluss mit der zentralen Steuereinheit (190) verbunden ist.

15. Labormaßstab-Ozonolyseapparatur (100) nach Anspruch 4 und 13, **dadurch gekennzeichnet, dass** das Temperatureinstellmittel (152) des zweiten Reaktors (150) durch einen entsprechenden elektrischen Anschluss mit der zentralen Steuereinheit (190) verbunden ist.

16. Labormaßstab-Ozonolyseapparatur (100) nach Anspruch 14 und 15, **dadurch gekennzeichnet, dass** sie ferner ein sekundäres Temperatureinstellmittel I (185) umfasst, welches in Wärmeübertragungsbeziehung mit dem Temperatureinstellmittel (132) des ersten Reaktors (130) und/oder dem Temperatureinstellmittel (152) des zweiten Reaktors (150) steht und durch einen entsprechenden elektrischen Anschluss mit der zentralen Steuereinheit (190) verbunden ist.

17. Labormaßstab-Ozonolyseapparatur (100) nach Anspruch 1, **dadurch gekennzeichnet, dass** das Vereinigungselement (120) mit einer Endplatte am Einlass davon versehen ist, durch welchen Ozon in den Strömungsweg geführt wird, wobei die Endplatte Perforationen aufweist, um die Größe der einzuführenden Ozonblasen zu verringern.

18. Verfahren im Labormaßstab zur Durchführung einer Ozonolysereaktion einer in einem Lösungsmittel gelösten Substanz, umfassend folgende Schritte:
(i) Zuführen einer bestimmten Menge einer einer Ozonolysereaktion zu unterziehenden gelösten Substanz in einen Strömungsweg mittels einer Förderpumpe (102) bei einem im Wesentlichen gleichmäßigen Volumendurchsatz;
(ii) Zuführen von Ozon durch ein Abgabeventil (112) in den Strömungsweg in einem Abschnitt, der hinter dem die Substanz zuführenden Abschnitt angeordnet ist, in Form von Mikroblasen, wobei das Ozon in situ durch Elektrolyse hergestellt wird;
(iii) Leiten der gelösten Substanz durch eine Reaktoreinheit, die die erste und die zweite Reaktorzone umfasst, die in einem hinter der Ozonzuführposition angeordneten Abschnitt des Strömungswegs angeordnet sind;
(iv) Zuführen eines für die Vollendung der Ozonolysereaktion benötigten Additivs in den Strömungsweg hinter der ersten Reaktorzone der Reaktoreinheit;
(v) Aufrechterhalten des Drucks der Reaktion in einem bestimmten Druckbereich mittels eines im Strömungsweg hinter der Reaktoreinheit angeordneten Druckeinstellmittels (160);
(vi) Sammeln des in der zweiten Reaktorzone der Reaktoreinheit erzeugten Produkts in einem mit dem Ende des Strömungswegs verbundenen Produktbehälter (180), wobei das gesamte Innenvolumen gemessen entlang des Strömungswegs, auch einschließlich des Volumens der Reaktoreinheit, höchstens 50 cm³ beträgt.

19. Verfahren im Labormaßstab zur Durchführung einer Ozonolysereaktion nach Anspruch 18, **dadurch gekennzeichnet, dass** das Ozon diskontinuierlich in den Strömungsweg zugeführt wird.

20. Verfahren im Labormaßstab zur Durchführung einer Ozonolysereaktion nach Anspruch 18, **dadurch gekennzeichnet, dass** die Temperatur der gelösten Substanz in eine vorgeschriebene Temperatur der in der ersten Reaktorzone der Reaktoreinheit durchzuführenden Reaktion umgeändert wird.

21. Verfahren im Labormaßstab zur Durchführung einer Ozonolysereaktion nach Anspruch 20, **dadurch gekennzeichnet, dass** eine Reaktion, die zur Zersetzung oder Stabilisierung des Zwischenprodukts, das durch die in der ersten Reaktorzone durchgeführte Reaktion gebildet wurde, führt, in der zweiten Reaktorzone der Reaktoreinheit durchgeführt wird.

22. Verfahren im Labormaßstab zur Durchführung einer Ozonolysereaktion nach Anspruch 21, **dadurch gekennzeichnet, dass** die Temperatur des in der ersten Reaktorzone gebildeten Zwischenprodukts und die Temperatur des in den Strömungsweg zugeführten Additivs in der zweiten Reaktorzone der Reaktoreinheit in eine vorgeschriebene Temperatur der in der zweiten Reaktorzone der Reaktoreinheit durchzuführenden Reaktion umgeändert werden.

## Revendications

1. Appareil d'ozonolyse de laboratoire de type à écoulement pour exécuter une réaction d'ozonolyse d'une substance donnée, l'appareil (100) comprenant un réservoir de liquide (104), une pompe d'alimentation (102) sous la forme d'une pompe à liquide générant un débit volumique constant, un élément d'union (120) avec deux entrées et une sortie, une unité de réacteur et un moyen d'ajustement de pression (160), tous connectés dans un chemin d'écoulement, l'appareil (100) comprenant en outre une source d'ozone (110) et une soupape de distribution (112) transmettant un courant de gaz uniquement dans un seul sens et étant installée entre la source d'ozone (110) et l'une des entrées de l'élément d'union (120), dans lequel le moyen d'ajustement de pression (160) est agencé après l'unité de réacteur et est prévu avec une commande électrique, **caractérisé en ce que** le réservoir de liquide (104) contient au moins la substance, sous la forme d'un soluté, devant être soumise à la réaction d'ozonolyse et l'unité de réacteur consiste en une première et une deuxième zones de réacteur étant différentes quant à leur fonction, dans lequel la sortie de la première zone de réacteur est connectée à l'entrée de la deuxième zone de réacteur dans le chemin d'écoulement et une admission de substance est insérée dans le chemin d'écoulement entre les zones de réacteur, et dans lequel la source d'ozone (110) est prévue comme une source d'ozone générant de l'ozone in situ par électrolyse et dans lequel le volume intérieur total mesuré le long du chemin d'écoulement entre la pompe d'alimentation (102) et le moyen d'ajustement de pression (160) est au plus 50 cm³_{.}

2. Appareil d'ozonolyse de laboratoire (100) selon la revendication 1, **caractérisé en ce que** les zones de réacteur de l'unité de réacteur sont prévues comme un premier et un deuxième réacteurs (130, 150) physiquement séparés.

3. Appareil d'ozonolyse de laboratoire (100) selon la revendication 2, **caractérisé en ce que** le premier réacteur (130) est prévu avec un moyen d'ajustement de température (132) en relation de transfert de chaleur avec le réacteur (130).

4. Appareil d'ozonolyse de laboratoire (100) selon la revendication 2, **caractérisé en ce que** le deuxième réacteur (150) est prévu avec un moyen d'ajustement de température (152) en relation de transfert de chaleur avec le réacteur (150).

5. Appareil d'ozonolyse de laboratoire (100) selon la revendication 2, **caractérisé en ce que** l'admission de substance est formée comme un élément d'union (140) avec deux entrées et une sortie, dans lequel la sortie du premier réacteur (130) est connectée à une des entrées de l'élément d'union (140), l'entrée du deuxième réacteur (150) est connectée à la sortie de l'élément d'union (140) et un deuxième réservoir de liquide (174) est connecté à l'autre entrée de l'élément d'union (140) par l'intermédiaire d'une deuxième pompe d'alimentation (172), dans lequel le deuxième réservoir de liquide (174) contient au moins un additif nécessaire pour la réalisation de la réaction d'ozonolyse.

6. Appareil d'ozonolyse de laboratoire (100) selon la revendication 5, **caractérisé en ce que** le premier réacteur (130) est prévu sous la forme d'un réacteur microfluidique (130') ayant une entrée, une sortie et un canal de réaction (325) pour recevoir des réactions chimiques, le réacteur microfluidique (130') comprenant un canal de réaction (325) scellé préparé dans la surface d'une tôle de réacteur (320) par façonnage à froid et scellé par un élément de fermeture (330) pressé sur ladite surface de la tôle de réacteur (320) et une unité de commande de température (350) agencée en contact avec une surface de l'élément de fermeture (330) opposée au canal de réaction (325) et étant en relation de transfert de chaleur avec le canal de réaction (325) par l'intermédiaire de l'élément de fermeture (330).

7. Appareil d'ozonolyse de laboratoire (100) selon la revendication 6, **caractérisé en ce que** l'élément d'union (120) est formé comme un élément intégral du canal de réaction (325).

8. Appareil d'ozonolyse de laboratoire (100) selon la revendication 6, **caractérisé en ce que** le moyen d'ajustement de température (132) est mis à disposition par l'unité de commande de température (350).

9. Appareil d'ozonolyse de laboratoire (100) selon la revendication 2, **caractérisé en ce que** le deuxième réacteur (150) est prévu sous la forme d'un réacteur microfluidique (130') ayant la même construction que celle du premier réacteur (130).

10. Appareil d'ozonolyse de laboratoire (100) selon la revendication 2, **caractérisé en ce que** le deuxième réacteur (150) est prévu sous la forme d'une colonne garnie avec une charge réactive, dans lequel la charge contient un additif nécessaire pour la réalisation de la réaction d'ozonolyse.

11. Appareil d'ozonolyse de laboratoire (100) selon la revendication 1, **caractérisé en ce que** la source d'ozone (110) est prévue sous la forme d'au moins une cellule d'électrolyse (110') générant de l'ozone sous pression asymétrique.

12. Appareil d'ozonolyse de laboratoire (100) selon la revendication 11, **caractérisé en ce que** la cellule d'électrolyse (110') générant de l'ozone comprend
- une cathode (13) ;
- une anode (16) comprenant un mélange d'oxyde de plomb (IV) et de polytétrafluoroéthylène ;
- une membrane (15) agencée entre la cathode (13) et l'anode (16) ; et
- un premier support d'électrode (17) électro-conducteur perméable aux liquides et aux gaz en contact avec un côté de l'anode (16) situé à l'opposé de la membrane (15), ledit côté du support d'électrode (17) ayant une surface recouverte avec une couche contenant du platine, dans lequel le matériau de l'anode (16) est un mélange préparé par moulage sous haute pression de grains d'oxyde de plomb (IV) de taille colloïdale et de filaments de polytétrafluoroéthylène ayant une dimension d'au plus 1 mm.

13. Appareil d'ozonolyse de laboratoire (100) selon la revendication 1, **caractérisé en ce qu'**il comprend en outre une unité de commande centrale (190) qui est connectée à la soupape de distribution (112), à la pompe d'alimentation (102) et au moyen d'ajustement de pression (160) par l'intermédiaire de connexions électriques appropriées.

14. Appareil d'ozonolyse de laboratoire (100) selon les revendications 3 et 13, **caractérisé en ce que** le moyen d'ajustement de température (132) du premier réacteur (130) est connecté avec l'unité de commande centrale (190) par l'intermédiaire d'une connexion électrique appropriée.

15. Appareil d'ozonolyse de laboratoire (100) selon les revendications 4 et 13, **caractérisé en ce que** le moyen d'ajustement de température (152) du deuxième réacteur (150) est connecté avec l'unité de commande centrale (190) par l'intermédiaire d'une connexion électrique appropriée.

16. Appareil d'ozonolyse de laboratoire (100) selon les revendications 14 et 15, **caractérisé en ce qu'**il comprend en outre un moyen d'ajustement de température secondaire (185) qui est en relation d'échange de chaleur avec le moyen d'ajustement de température (132) du premier réacteur (130) et/ou le moyen d'ajustement de température (152) du deuxième réacteur (150), et est connecté avec l'unité de commande centrale (190) par l'intermédiaire d'une connexion électrique appropriée.

17. Appareil d'ozonolyse de laboratoire (100) selon la revendication 1, **caractérisé en ce que** l'élément d'union (120) est prévu avec une tôle d'extrémité au niveau de l'entrée de celui-ci à travers laquelle l'ozone est alimenté dans le chemin d'écoulement, ladite tôle d'extrémité ayant des perforations pour diminuer la taille des bulles d'ozone devant être introduites.

18. Procédé de laboratoire pour exécuter une réaction d'ozonolyse d'une substance dissoute dans un solvant, comprenant les étapes de
(i) admission d'une quantité donnée de substance étant dissoute destinée à être soumise à une réaction d'ozonolyse dans un chemin d'écoulement au moyen d'une pompe d'alimentation (102) à un débit volumique sensiblement constant ;
(ii) alimentation d'ozone par une soupape de distribution (112) dans le chemin d'écoulement dans une section située après la position d'admission de substance sous la forme de microbulles, ledit ozone étant produit in situ par électrolyse ,
(iii) conduite de la substance dissoute à travers une unité de réacteur comprenant une première et une deuxième zones de réacteur agencée dans une section du chemin d'écoulement située après la position d'alimentation d'ozone ;
(iv) admission d'un additif nécessaire pour la réalisation de la réaction d'ozonolyse dans le chemin d'écoulement après la première zone de réacteur de l'unité de réacteur ;
(v) maintien de la pression de la réaction dans une plage de pression donnée au moyen d'un moyen d'ajustement de pression (160) agencé dans le chemin d'écoulement après l'unité de réacteur ;
(vi) récupération du produit généré dans la deuxième zone de réacteur de l'unité de réacteur dans un réceptacle de produit (180) connecté à l'extrémité du chemin d'écoulement, dans lequel le volume intérieur total mesuré le long dudit chemin d'écoulement, incluant également le volume de l'unité de réacteur, est au plus 50 cm³.

19. Procédé de laboratoire pour exécuter une réaction d'ozonolyse selon la revendication 18, **caractérisé en ce que** l'ozone est admis dans le chemin d'écoulement de façon intermittente.

20. Procédé de laboratoire pour exécuter une réaction d'ozonolyse selon la revendication 18, **caractérisé en ce que** la température de la substance dissoute est amenée à une température prescrite de la réaction devant être exécutée dans la première zone de réacteur de l'unité de réacteur.

21. Procédé de laboratoire pour exécuter une réaction d'ozonolyse selon la revendication 20, **caractérisé en ce qu'**une réaction résultant en la décomposition ou la stabilisation de l'intermédiaire produit par la réaction exécutée dans la première zone de réacteur est exécutée dans la deuxième zone de réacteur de l'unité de réacteur.

22. Procédé de laboratoire pour exécuter une réaction d'ozonolyse selon la revendication 21, **caractérisé en ce que** la température de l'intermédiaire produit dans la première zone de réacteur et la température de l'additif admis dans le chemin d'écoulement sont amenées dans la deuxième zone de réacteur de l'unité de réacteur à une température prescrite de la réaction devant être exécutée dans la deuxième zone de réacteur de l'unité de réacteur.
